# FASCICULE DE BREVET EUROPEEN

(11) **EP 1 042 363 B1**
(45) Date de publication et mention de la délivrance du brevet: **21.06.2006**
(21) Numéro de dépôt: 98959984.0
(22) Date de dépôt: 14.12.1998
(51) Int. Cl.: C07K 14/16, A61K 39/00

(54) **NOUVEAUX IMMUNOGENES ANTI-HIV (TOXOIDES), PROCEDES DE PREPARATION ET APPLICATION A LA PREVENTION ET AU TRAITEMENT DU SIDA**
NEUE ANTI-HIV IMMUNOGENE (TOXOIDE), HERSTELLUNGSVERFAHREN UND VERWENDUNGEN ZUR BEHANDLUNG UND VORBEUGUNG VON AIDS
NOVEL ANTI-HIV IMMUNOGENS (TOXOIDS), PREPARATION METHODS AND USE FOR PREVENTING AND TREATING AIDS

(30) Priorité: 26.12.1997 FR 9716589
(43) Date de publication de la demande: 11.10.2000
(73) Titulaire: NEOVACS, 75106 Paris (FR)
(72) Inventeur: ZAGURY, Jean-François, F-75003 Paris (FR); RAPPAPORT, Jay, Balacynwyd, PA 19004 (US); CARCAGNO, Miguel, AR-1427 Buenos Aires (AR)
(74) Mandataire: Catherine, Alain
(86) Numéro de dépôt international: PCT/FR1998/002727
(87) Numéro de publication internationale: WO 1999/033872

(56) Documents cités:
- WO-A-86/06414
- WO-A-91/18454
- WO-A-94/15634
- WO-A-96/27389
- RAPPAPORT J. ET AL.: "HIV-1 Tat toxoid: rationale for a Tat vaccine and results of a phase I clinical trial." JOURNAL OF NEUROVIROLOGY, vol. 4, no. 3, 3 juin 1998, page 364 XP002078560

## Description

Le Sida est induit par l'infection par le virus VIH-1. Ce virus est composé de plusieurs protéines. Parmi ces protéines, Tat est, avec la protéine Nef, produite la plus précocement dans le cycle viral.

Pour lutter contre les effets de la protéine Tat plusieurs approches ont été proposées telles des approches génétiques pour bloquer l'action transcriptionnelle de Tat. Ainsi des essais cliniques ont eu lieu avec des anti-sens (société Hybridon), des expériences in vitro ont montré la faisabilité de ribozymes ou encore de leurres utilisant l'ARN de la région TAR (site de liaison du Tat à l'ARNm).

Des molécules pharmacologiques inhibitrices de l'effet transcriptionnel de Tat ont aussi été décrites.

Toutes ces méthodes visent à bloquer la réplication du virus en bloquant l'action transcriptionnelle de Tat.

Des méthodes immunologiques ont été aussi proposées de très longue date.

Pour une immunisation spécifique contre la protéine Tat native WO-A-91/18454 rapporte l'utilisation de polypeptides de protéines rétrovirales, incluant la protéine Tat native, obtenues par coupures protéolytiques, synthèse chimique ou recombinaison génétique, comme immunogènes pour prévenir la réplication rétrovirale et l'activité cytotoxique contre en particulier les lymphocytes et les cellules nerveuses exercées par les protéines rétrovirales dont Tat en particulier. La toxicité des protéines natives ou leur fragments empêche cependant leur utilisation pour une immunisation.

WO-A-95/31999 rapporte l'utilisation de la protéine Tat native de VIH-1, de fragments de ladite protéine ou de polypeptides avec des délétions/substitutions comme immunogènes afin de bloquer la réplication du VIH-1 en bloquant la capture de la protéine Tat extracellulaire par les cellules non infectées. Malgré l'immunogénicité décrite, compte tenu des effets toxiques de la protéine native Tat en particulier, les désordres immunitaires qu'elle induit, et de l'absence d'anticorps neutralisants par immunisation avec les immunogènes décrits dans ce brevet, il y a effectivement un besoin de développer de nouveaux immunogènes Tat inactivés (qui ont perdu tous les effets nocifs de la protéine Tat native) capables d'induire une réponse immunitaire humorale et cellulaire.

WO-A-96/27389 rapporte l'utilisation de nouveaux produits, des toxoïdes de Tat et de protéines régulatrices rétrovirales comme immunogènes capables d'induire une réponse immunitaire contre la protéine Tat native et de prévenir ou réparer ses effets irrymunosuppressrts. Ces toxoîdes, produits inactifs mais immunogéniques, ont été préparés par traitement chimique au formaldéhyde de la protéine native ou de segments dérivés de cette protéine. Mais l'inactivation à l'aide d'un aldéhyde est délicate. Or pour une production industrielle, il faut des résultats réguliers, notamment pour l'inactivation. Les agences de santé préfèrent en outre des produits de structure constante et bien définie pour autoriser la mise sur le marché. Un produit idéal devrait en outre avoir une bonne stabilité dans le temps.

C'est pourquoi la présente demande a pour objet de nouveaux toxoïdes et une nouvelle méthode simple et efficace pour fabriquer ces toxoïdes notamment à partir des protéines de régulation du VlH-1 qui contribuent à l'effet immunosuppressif du VIH-1.

La stratégie des toxoïdes a de gros avantages sur les substitutions/délétions d'acides aminés: les régions de la protéine Tat native responsables des effets pléïomorphes de Tat ne sont pas bien identifiées et il est difficile de prédire a priori les substitutions/délétions qui assurent une innocuité totale à la préparation. Par exemple la mutations du résidu C25 en G abroge l'effet transactivateur de Tat sans supprimer - son effet immunosuppressif. D'autre part, les substitutions/délétions peuvent altérer fortement la structure de la molécule modifiée par rapport à la molécule native (antigènes linéaires et conformationnels) et ainsi empêcher le développement d'une réaction immunitaire efficace contre la molécule native. Ce dernier point est particulièrement important pour la génération d'anticorps neutralisants contre la protéine native.

La présente demande a donc pour objet une protéine de régulation virale ou un fragment de 8 à 110 acides aminés de protéine de régulation virale caractérisés en ce qu'ils sont carboxyméthylés, par réaction avec l'acide iodoacétique ou par réaction avec l'iodoacétamide.

Dans des conditions préférentielles de mise en oeuvre de l'invention, la protéine ou le fragment ci-dessus provient du virus VIH-1, VIH-2, HTLV-1 ou HTLV-2 et notamment du virus VIH 1 ou VlH 2.

Dans d'autres conditions préférentielles de mise en oeuvre de l'invention la protéine ou fragment ci-dessus est une protéine de régulation virale ou un fragment de protéine de régulation virale.

Dans encore d'autres conditions préférentielles de mise en oeuvre de l'invention la protéine ou fragment ci-dessus provient de TAT notamment de VIH-1.

La présente demande a également pour objet un procédé de préparation d'une protéine ou d'un fragment tel que défini ci-dessus, caractérisé en ce que l'on soumet ladite protéine de régulation virale ou ledit fragment à une carboxymëihylation, pour obtenir le composé attendu que l'on isole.

Selon l'invention, les protéines ou fragments qui se comportent comme des toxines sur le système immunitaire, que ce soit Tat ou Nef par exemple sont modifiés par la carboxyméthytation.Cette modification chimique conduit à de nouveaux protéines ou fragments qui sont inactifs biologiquement mais conservent leur immunogènicité. En d'autres termes, ces protéines ou fragments qui se comportent comme des toxines sur le système immunitaire sont reconnus par des anticorps produits contre les protéines ou fragments carboxyméthylés selon t'invention. Ces protéines ou fragments carboxyméthylés conservent des propriétés immunogènes suffisantes pour créer des anticorps neutralisant ou bloquant ladite protéine native et en même temps ont perdu au moins 90 %, de préférence au moins 95 %, notamment au moins 99 %, des propriétés biologiques toxiques de ladite protéine native c'est à dire ses propriétés biologiques habituafies bien connues.

Le composé immunogène carboxyméthylé selon l'invention peut être constitué de la totalité ou d'un fragment de 8 à 110 acides aminés de la protéine notamment de régulation et peut comporter, comme c'est bien connu de l'homme de l'art, une ou plusieurs modifications dans les acides aminés de cette protéine ou fragment telles que des délétions, substitutions, additions, ou fonctionnaüsations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés. Les modifications doivent généralement concerner moins de 30 % des acides aminés, de préférence moins de 20 % et tout particulièrement moins de 10 %.

Un fragment peut comporter de 8 à 110 acides aminés par exemple, de préférence de 12 à 60 acides aminés et notamment de 12 à 40 acides aminés. Un tel fragment peut comporter aussi du ou des côtés C ou N terminal de 1 à 5 acides aminés supplémentaires c'est-à-dire différents du segment d'origine. Un fragment doit en outre comporter une cystéine au moins pour pouvoir faire l'objet de la carboxyméthylation

De manière générale, en ce qui concerne les modifications, l'homologie ou la similitude entre l'immunogène modifié et la protéine ou partie de protéine native, ainsi que les dimensions du composé immunogène, de même que les modalités d'utilisation, ou de couplage du composé immunogène selon l'invention à une protéine immunogène telle que le toxoïde tétanique, on peut en particulier se référer à WO-A-86/06 414 ou à EP-A-0.220.273 ou encore à PCT/US.86/00831, équivalents, dont l'enseignement est incorporé ici par référence.

Les composés immunogènes selon l'invention dérivant de protéines de régulation seront appelées parfois ci-après dans la partie expérimentale « toxoïdes viraux ».

En effet, .tout comme les toxoïdes bactériens classiques, ils sont dépourvus de toxicité propre, mais sont cependant capables de provoquer une immunisation par administration chez un sujet.

Afin de vérifier que la protéine de régulation native est bien reconnue par des anticorps dirigés contre ladite protéine de régulation modifiée, on peut par exemple vérifier immunologiquement par Elisa en présence d'anticorps spécifiques, la formation de complexes antigènes-anticorps comme on le verra ci-après dans la partie expérimentale,

Afin de déterminer si les propriétés immunogènes de la protéine de régulation ont été suffisamment conservées pour créer des anticorps neutralisant ladite protéine native, on peut par exemple immuniser des mammifères (lapins, rats, souris) à l'aide d'un composé immunogène selon l'invention et vérifier que tes anticorps produits neutralisent les activités toxiques du Tat.

Afin de déterminer si la protéine de régulation modifiée a perdu au moins de 90% de ses propriétés biologiques toxiques, on peut par exemple en ce qui concerne le Tat étudier l'effet du Tat inactivé sur l'immunosuppression des cellules T ou sur la néoangiogénèse.

L'inactivation de la protéine de régulation Tat est vérifiée par exemple par le « Tat Rescue Assay » utilisant un mutant de HIV non infectieux Tat déficient cultivé sur la lignée cellulaire HL-1 dont la réplication dépend d'un apport exogène de Tat natif.

Le composé immunogène selon l'invention peut dériver notamment d'une quelconque des protéines de régulation des virus V1H-9, VIH-2, HTLV-1 ou HTLV-2 et notamment Nef, Rev et de préférence Tat des virus VIH-1 et VIH-2.

On peut aussi citer la protéine Tax de HTLV-1 ou HTLV-2.

La présente demande a également pour objet un procédé de préparation d'uns protéine ou d'un fragment tel que défini ci-dessus, caractérisé en ce que l'on soumet ladite protéirie de régulation virale ou ledit fragment à une carboxyméthylation, par réaction avec l'acide iodoacétique ou par réaction avec l'iodoacétamide. pour obtenir le composé attendu que l'on isole si désiré.

La réaction de carboxyméthylation permet de modifier les groupements thiols (groupement sulfhydryl) présents au niveau des résidus cystéines de l'enchaînement en acides aminés. Ces groupements réagissent avec l'acide iodoacétique ou l'iocloacétamide par une réaction de S-carboxyméthylation ou S-carboxyamidométhyiation, respectivement

Par exemple, la protéine Tat possède 7 cystéines. Ces cystéines participent à la formation de ponts disulfure inter et intra-chaînes et contribuent à la formation d'oligomères.

Le produit de la réaction est dans chaque cas un résidu S-carboxyméthylcystéinyl ou S-carboxyméthylamidocystéinyl.

La réaction de carboxyméthylation peut aussi être réalisée avec d'autres agents chimiques comme l'acide performique, l'acide 3-bromopropionique, l'éthylénéimine, le bromure de (2-bromoéthyl) triméthylammonium, le 2-bromoéthane sulfonate, la 1,3-propanesulfone etc....

Dans des conditions préférentielles de mise en oeuvre du procédé ci-dessus décrit, ladite protéine ou ledit fragment de départ se présente sous forme fusionnée à un marqueur (FP) lorsqu'on le soumet à la carboxyméthylation.

Les protéines ou fragments de départ du procédé sont des produits connus, décrits dans la littérature, par exemple pour Tat de VIH-1 par Frankel A.D. et al. (Cellular uptake of the tat protein from human immunodeficiency virus, Cell, 1988, 55 : 1189-93) ou pour Nef de VIH-1, par Azad A.A. et al. (Large-scale production and characterization of recombinant human immunodeficiency virus type 1 Nef, J. Gen. Virol. 1994, 75 : 651-55), ou peuvent être préparés de manière conventionnelle.

On peut en particulier préparer les protéines ou fragments de départ ci-dessus par :
1) Synthèse par génie génétique ou par synthèse biochimique ;
2) Purification

Par génie génétique, on peut purifier les protéines produites par chromatographie d'affinité en utilisant par exemple des anticorps dirigés contre la protéine ou un de ses fragments ; on peut aussi synthétiser la protéine fusionnée à un marqueur (FP) qui servira à l'accrochage à une colonne d'affinité.

Dans d'autres conditions préférentielles de mise en oeuvre du procédé ci-dessus décrit, lorsque la protéine ou fragment est fusionné à un marqueur (FP), il est soumis à :
- une concentration, par exemple, par ultrafiltration ;
- un désalage, par exemple, par gel filtration ;
- un traitement au bromure de cyanogène ou l'entérokinase pour cliver la protéine de fusion et libérer ainsi la protéine ou fragment ;
- une concentration et diafiltration ;
- une chromatographie par échange cationique ;
- une concentration par ultrafiltration, suivie par une filtration sur gel d'exclusion.

La réaction au bromure de cyanogène ci-dessus permet de cliver les ihioéthers. L'action du bromure de cyanogène sur les molécules polypeptidiques est sélective en effectuant un clivage au niveau des résidus méthionine existants. Cette réaction aboutit à la formation de 2 fragments polypeptides par résidu méthionine. Cette réaction peut être avantageusement couplée avec la réaction de carboxyméthylation précédemment décrite mais elle n'est pas nécessaire à l'inactivation.

Dans d'autres conditions préférentielles de mise en oeuvre du procédé ci-dessus décrit, on prépare la protéine ou le fragment attendu(e) couplé(e) à un composé permettant sa purification, par exemple à un fragment peptidique contenant plusieurs histidines, de préférence en séquence continue de 4, 5, notamment 6 histidines ou plus permettant la fixation à une colonne de Nickel. Dans la mesure où la présence de ce composé n'induit pas de toxicité et ne modifie pas défavorablement l'immunogénicité de la protéine ou de fragment, il n'est pas nécessaire de le cliver après purification. Toutefois, dans des conditions de réalisation préférée, on clive ce composé pour réliminer.

Les composés objet de la présente invention possèdent de très intéressantes propriétés pharmacologiques. Ils sont inactifs biologiquement vis à vis des fonctions qu'ils exercent habituellement comme les effets imrnunosuppressifs, les effets transactrveurs du promoteur du VtH-1, ou les effets oxydatifs pour Tat.

Ils sont immunogènes chez la souris mais aussi chez l'homme. Ils permettent notamment l'induction d'une réponse immunitaire humorale avec anticorps neutralisants et d'une réponse immunitaire cellulaire.

Ces propriétés sont illustrées ci-après dans la partie expérimentale. Elles justifient l'utilisation des protéines ou fragments ci-dessus décrits ainsi que de leurs sels d'addition avec les acides pharmaceutiquement acceptables, à titre de médicament.

C'est pourquoi l'invention a aussi pour objet une protéine ou un fragment carboxyméthylé ci-dessus, pour son utilisation dans une méthode de traitement thérapeutique du corps humain ou animal, c'est-à-dire à titre de médicament

Les médicaments selon la présente invention trouvent leur emploi par exemple dans le traitement tant curatif que préventif des effets nocifs provoqués par une surproduction d'une protéine de régulation d'un virus, notamment d'un rétrovirus VIH-1. VlH-2, HTLV-1 ou HTLV-2.

Les protéines Tat et Nef ont des régions relativement bien conservées. Cependant, le cas échéant, on pourra immuniser le même sujet avec des toxoîdes préparés à partir de souches variables, notamment pour s'adapter aux variants géographiques de l'épidémie.

Les composés immunogènes selon l'invention peuvent être utilisés comme suit :

On administre à un patient un composé immunogène selon la présente invention, par exemple par voie sous-cutanée ou intramusculaire, en quantité suffisante pour être efficace sur le plan thérapeutique, à un sujet ayant besoin d'un tel traitement. La dose administrée peut aller par exemple de 50 à 1000 µg par voie intra musculaire sous forme d'énnulsion e/h, une fois par mois pendant trois mois, puis périodiquement en fonction du taux des anticorps sériques induits, par exemple tous les 2-6 mois.

A titre de médicaments, les protéines ou fragments carboxyméthylés ci-dessus peuvent donc être incorporés dans des compositions pharmaceutiques destinées à la voie orale et notamment parentérale.

Une composition selon l'invention peut être administrée par n'importe quelle voie conventionnelle en usage dans le domaine des vaccins, en particulier par voie sous-cutanée, par voie intramusculaire, par voie intraveineuse ou par voie orale. L'administration peut avoir lieu en dose unique ou répétée une ou plusieurs fois après un certain intervalle de temps.

L'invention a aussi pour objet les compositions pharmaceutiques qui renferment au moins un composé précité, à titre de principe actif.

Dans ces compositions, le principe actif est avantageusement présent à des doses physiologiquement efficaces ; les compositions précitées renferment notamment une dose immunogène efficace d'au moins un principe actif ci-dessus. Le composé immunogène peut être conditionné seul ou mélangé à un excipient pharmaceutiquement acceptable tel qu'un adjuvant.

Ces compositions pharmaceutiques peuvent être, notamment liquides et se présenter sous les formes pharmaceutiques couramment utilisées en médecine humaine pour les vaccins, comme par exemple les préparations injectables notamment sous forme d'émulsion ; elles sont préparées selon les méthodes usuelles. Le ou les principes actifs peuvent y être incorporés à des excipients habituellement employés dans ces compositions pharmaceutiques, tels que les véhicules aqueux, le phosphate de calcium, l'alun...

L'invention a notamment pour objet à titre de compositions pharmaceutiques.
a) Une composition pharmaceutique comprenant à titre d'agent préventif ou curatif un toxoïde viral ou un fragment ou analogue de protéine de régulation, notamment du Tat, selon l'invention.
b) Une composition pharmaceutique comprenant à titre d'agent préventif ou curatif des anticorps anti-Tat produits à partir d'organismes immunisés contre ladite protéine ou ses fragments F(ab')2 ou Fab, selon l'invention.

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

En outre l'invention a pour objet un kit comprenant une composition pharmaceutique vaccinale qui en plus du principe actif (par exemple Tat-toxoïde ou ses dérivés ou anticorps anti-Tat) peut comprendre un adjuvant et/ou un autre immunogène à propriétés anti-rétrovirus.

On peut citer par exemple comme autre immunogène la protéine d'enveloppe GP 160, notamment la glycoprotéine tansmembranaire GP 10 de VIH-1 ou leurs fragments connus de l'état de la technique, la protéine Gag de la capside virale, ou encore la protéine Pol.

L'invention a enfin pour objet l'utilisation d'une protéine ou fragment carboxyméthylé ci-dessus, pour l'obtention d'un médicament destiné à une utilisation en tant qu'immunogène.

Les exemples qui suivent illustrent la présente demande.

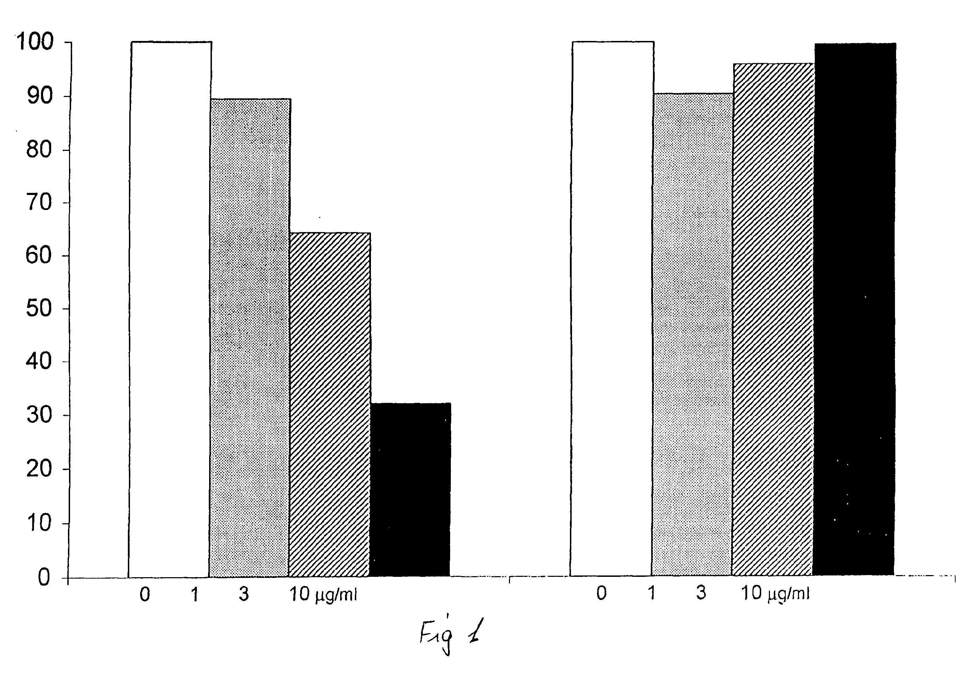

La figure 1 représente les résultats de l'évaluation des effets du Tat natif comparés à ceux du produit de l'exemple 1 sur la prolifération de lymphocytes normaux stimulés par un antigène de rappel.

### Préparation A.

On cultive 2 ml de la bactérie déposée à la Collection Nationale de Cultures de Microorganismes de Paris le 26 Décembre 1997 sous le N° I-1964 qui est une bactérie E coli dans laquelle on a inséré par transfection un plasmide recombinant contenant un gène codant pour un polypeptide comportant un fragment constitué de 6 histidines, associé au gène de la protéine Tat, dans 400 ml d'un milieu de culture de base (extrait de levure 5g/l, tryptone 1g/l, chlorure de sodium 1g/l) et 40 ml d'une solution 1 (CaCl₂2H₂O : 0,175 g/l, MgSO₄7H₂O : 5,9 g/l, glucose : 6 g/l). On incube ainsi la culture à 30°C pendant 10 heures à 250 tr/mn.

La préfermentation ci-dessus est suivie d'une fermentation dans des conditions analogues, en maintenant le niveau d'oxygène dissous à un niveau de 70 % de la saturation par régulation de l'agitation. Lorsque la densité optique mesurée à 650 nm atteint la valeur de 6, on ajoute 100 ml d'une solution stérile à 3,75 % d'IPTG (isopropyle β-d-thiogalactopiranoside) dans de l'eau désionisée. En même temps, on supplémente le milieu avec une solution stérile d'extrait de levure à 25 %. On ajoute alors une solution comprenant MgSO₄7H₂O : 8,5 g/l, glucose : 300 g/l, (NH₄)₂SO₄ : 106 g/l, oligo-éléments, en maintenant la concentration de glucose au delà de 2 g/l. On récolte alors la masse bactérienne 4 heures après l'introduction de l'IPTG. On procède à une diafiltration contre un tampon tris 0,1 M, NaH₂PO₄ 0,1 M, ditriothéitol 1 Mm, pH 8,0, par filtration à contre-courant (seuil 0,3 µm).

Trois litres du produit brut ci-dessus sont lysés par 4 passages à une surpression de 500 bars. Le lysat est alors centrifugé pendant 15 mn à 4°C et 5000 tr/mn. On ajoute alors au surnageant une quantité suffisante d'urée pour obtenir une solution 8 M et on ajuste le pH à 8.

On procède alors à une purification par chromatographie d'affinité sur une colonne nickel-NTA agarose (Qiagen) préalablement équilibrée avec 600 ml de tampon A (urée 8M, NaH₂PO₄ 0,1 M, tris-HCl 0,1 M, DTT 1 Mm, pH 8,0). Une fois chargée, la colonne a été lavée avec 250 ml de tampon A. On élue alors la protéine en utilisant un gradient discontinu pour obtenir la protéine fusionnée attendue.

On concentre la solution ainsi obtenue sur membrane Amicon à seuil de coupure de 3000 daltons pour obtenir une concentration finale de 10 mgimi. On obtient ainsi un éluat brut renfermant la protéine recombinante fusionnée de VIH-1 Tat attendue.

On a produit ainsi une protéine de fusion Tat fusionnée génétiquement avec un fragment peptidique riche en histidines qui permet son accrochage au nickel en vue d'une purification.

L'éluat ainsi obtenu sera soumis à la réaction de carboxyméthylation.

### Exemple 1: Préparation de toxoïde Tat

### Stade A : Carboxyméthylation

On ajuste une solution de la protéine TAT fusionnée à un fragment peptidique riche en histidine préparée à la préparation 1 ci-dessus pour obtenir les concentrations suivantes: Urée 8M, Tris HCl 0,3M, Ditriothéitol, 10 mM, pH 8,4.

Sous atmosphère inerte, on ajoute 2,6 g d'acide iodoacétique à 100 ml de la solution ci-dessus. Cette solution est incubée à 37°C à l'abri de la lumière et sous atmosphère inerte pendant 90 mn. La réaction est bloquée par addition de 1 ml de β-mercaptoéthanol à 98% et l'incubation est poursuivie pendant 60 minutes dans les mêmes conditions que ci-dessus.

La solution résultant de l'étape précédente est concentrée à l'aide d'un concentrateur Amicon (Cat#8400) sur membrane YM3 (seuil de 3000 D) jusqu'à une concentration de 10 mg/ml.

On la désale alors par passage dans une colonne Cellufine GH25 (MATREX) équilibrée à l'aide de 300 ml d'une solution d'urée 4M, 0,1 M HCl.

### Stade B -Purifications

L'éluat est alors ultrafiltré et traité au bromure de cyanogène pour cliver au niveau de la méthionine la partie amino terminale du produit carboxyméthylé. Du bromure de cyanogène est ajouté en excès sous atmosphère inerte, dans la proportion d'environ 50 moles de bromure de cyanogène par mole de méthionine. Cette solution est conservée dans un récipient clos pendant 24h à 37°C. L'excès de bromure de cyanogène est chassé par évaporation sous pression réduite.

La solution obtenue est alors concentrée, diafiltrée contre du tampon acide acétique-acétate de sodium 0,05M, pH 5 en utilisant un diafiltreur Amicon muni d'une membrane YM3 (seuil 3000 KD). La quantité de produit inactif obtenu en solution est de l'ordre de 450 mg.

Cette solution est filtrée sur une colonne échangeuse d'ions SP-Sépharose FF préalablement équilibrée avec 200 ml de tampon A et le produit est élué par un gradient de NaCl.

L'éluat est à nouveau concentré par diafiltration dans le même système Amicon, et la fraction obtenue est purifiée par gel filtration sur colonne de Sephacryl S (Pharmacia) équilibrée avec du tampon phosphate pH 7,4. Le produit final est filtré sur membrane à 0,22 um et stocké à 4° jusqu'à utilisation.

Ce produit a été soumis aux analyses ci-dessous, et a fait l'objet des tests pharmacologiques.

### Analyses.

- Quantité totale de produit inactivé dosée par le test de Bradford : 150 mg.
- Electrophorèse sur gel de polyacrylamide SulfoDodécylSulfate-PAGE, coloration argent (Phast System, Pharmacia, gel homogène à 20%) : bande unique avec un poids moléculaire compatible avec celui attendu. Pas d'autres bandes détectables à des poids supérieurs ou inférieurs.
- Electrophorèse par gradient isoélectrique (Phast System, Pharmacia, GEL IEF 3-9): une seule bande visible.
- Western Blot (Phast System, Pharmacia, anticorps monoclonal de l'hybridome 5G11) : une seule bande observée et absence de matériel agrégé ou dégradé.
- Activité biologique (induction du gène CAT dans la lignée HELA-HIV-1-LTR CAT): aucune activité détectable.
- Séquençage de la partie N-terminale en utilisant la technique standard de séquençage d'Edman avec un séquenceur Applied Biosystems (model 477A): les 20 premiers acides aminés obtenus coïncident avec la séquence attendue
   E-P-V-D-P-R-L-E-P-W-K-H-P-G-S-Q-P-K-T-A
- Endotoxines (test LAL selon le protocole USP XXlll): moins de 0,5 unités endotoxique par mg de protéine.
- Stérilité (selon le protocole USP XXIII): répond aux normes.
- Mesure du degré de substitution : en utilisant le réactif d'Ellman pour déterminer les groupements sulfhydriles libres par comparaison avec une courbe standard mesurant les cystéines. Comparant le Tat natif au Tat carboxyméthylé, on a trouvé que seulement 0,03 % des cystéines restaient actives dans le Tat carboxyméthylé, soit 1 cystéine active pour 3330 cystéines inactives. Un simple calcul montre que pour avoir une molécule Tat avec 7 cystéines actives dans ces conditions, il faudrait plusieurs kg de protéine Tat. L'inactivation est donc quasi-totale.

### Exemple 2. Préparation du toxoïde Nef.

Pour le clonage, ce sont des amorces Nef qui sont utilisées pour obtenir le gène Nef par RT-PCR à partir d'ARN de cellules infectées par le VIH-1. C'est ensuite le même plasmide avec le même segment fusogène riche en lysine qui permet l'accrochage sur la colonne de Nickel qui est utilisé. Le protocole de production et de purification est alors le même que dans l'exemple 1 mais la coupure est effectuée par action d'entérokinase et non de bromure de cyanogène.

Les résultats d'analyse sont similaires à ceux obtenus avec la protéine Tat.

### Exemple 3. Préparation du toxoïde IFNa.

Cette fois le plasmide n'utilise pas une protéine de fusion. La protéine est produite telle quelle par génie génétique sans segment fusogène, et est purifiée en utilisant une colonne de chromatographie à laquelle sont accrochés des anticorps monoclonaux anti-IFNa. Une fois éluée, la protéine est immédiatement inactivée selon le même protocole que celui décrit dans l'exemple 1.

Les résultats d'analyse sont identiques à ceux obtenus avec la protéine Tat avec un test biologique montrant une activité antivirale (virus VSV sur cellules MDBK) nulle : l'inactivation biologique est totale.

### ETUDE PHARMACOLOGIQUE

### 1) Absence d'effet immunosuppressif du toxoïde Tat.

Il existe un test simple montrant l'effet immunosuppressif de la protéine Tat native. Des cellules du sang périphérique (PBMCs) sont incubées en milieu sans sérum avec de la protéine Tat native ou du Tat toxoïde à des concentrations variant de 0 à 10 mg/ml. Après 2h de contact, du sérum est ajouté à la concentration finale de 10% et les cellules sont stimulées de la manière habituelle par du Staphylococcus Entérotoxine B (SEB) ou par un antigène de rappel Protein Purified Derived (PPD). La prolifération est évaluée après 3 jours SEB ou 5 jours PPD.

On voit que la prolifération est diminuée de manière dose-dépendante par le Tat natif alors qu'elle ne l'est pas par le toxoïde.

Sur la figure 1, les 4 barres de gauche correspondent au Tat natif et les 4 barres de droite correspondent au composé de l'exemple 1 (Tat toxoïde). Les chiffres en abscisse sont les concentrations de produit en µg/ml, respectivement 0, 1, 3, 10 µg/ml. Les ordonnées précisent la prolifération des cellules T en pourcentage. Le contrôle correspond à une concentration de Tat utilisé égale à 0. On voit que le Tat natif inhibe la prolifération cellulaire alors que le composé de l'exemple 1 n'a pas d'effet.

### 2) Immunogénicité du toxoïde Tat chez la souris, production d'anticorps neutralisants.

Du Tat toxoïde préparé selon l'exemple 1 a été utilisé pour immuniser des souris. Le protocole d'immunisation est celui classiquement utilisé : On injecte par voie intramusculaire (im) aux souris 100 µl d'émulsion (1:1) en adjuvant complet de Freund contenant 20 µg de produit au jour 0 avec rappel en adjuvant incomplet de Freund de 5 µg aux jours 21 et 35. Le sérum des souris est prélevé aux jours -2, 28 et 40 et les anticorps anti-Tat mesurés par ELISA sur des plaques sensibilisées avec de la protéine Tat recombinante native (non traitée chimiquement) ou avec du composé de l'exemple 1. Les sérums ont été testés à une dilution au 1/1000. Les résultats obtenus exprimés en densité optique sur 3 souris immunisées (1 à 3) et 3 souris non immunisées (4 à 6) sont présentés dans le tableau suivant :

| | | Tat natif | Tat toxoïde |
|---|---|---|---|
| Souris 1 | J -2 | 0,3 | 0,3 |
| | J 28 | 1,6 | 1,5 |
| | J 40 | 2 | 2,1 |
| Souris 2 | J -2 | 0,2 | 0,3 |
| | J 28 | 1,9 | 1,8 |
| | J 40 | 2,2 | 2,2 |
| Souris 3 | J -2 | 0,4 | 0,4 |
| | J 28 | 1,7 | 1,5 |
| | J 40 | 1,9 | 2,2 |
| Souris 4 | J-2 | 0,2 | 0,2 |
| | J 28 | 0, 3 | 0,2 |
| | J 40 | 0,3 | 0,3 |
| Souris 5 | J-2 | 0,4 | 0,4 |
| | J 28 | 0,4 | 0,3 |
| | J 40 | 0, 3 | 0,3 |
| Souris 6 | J -2 | 0,3 | 0,3 |
| | J 28 | 0,4 | 0,3 |
| | J 40 | 0,3 | 0,3 |

Ces résultats montrent que les souris immunisées par le toxoïde produisent des anticorps reconnaissant de la même façon la protéine native et le toxoïde.

Ces anticorps sont de plus neutralisants. Ceci a été établi à l'aide de 2 tests différents:

Le test d'inactivation de la protéine Tat. On ajoute à des cellules en culture de la lignée HELA-HIV-1-LTR-CAT, qui sont transfectées de manière stable avec un plasmide contenant le Long Terminal Repeat (LTR) du VIH-1 comme promoteur du gène Chloramphénicol Acétyl Transférase (CAT), de la protéine Tat native ou du toxoïde Tat, ou de la protéine Tat native préincubée 1 heure avec du sérum (dilution 1/50) de souris (J 0 ou J 40) immunisée ou non immunisée. La protéine Tat native à 5 µg/ml pénètre dans les cellules et va jouer son rôle transactivateur sur le LTR de VIH-1 et induire l'expression de la protéine CAT. Après 48H, les cellules sont lysées et la quantité de protéine CAT présente est mesurée par un test ELISA (Boehringer). Les résultats sont exprimés en densité optique:

| | | |
|---|---|---|
| | N° de la souris / jour | DO (mesure de protéine CAT) |
| Tat natif | | 1,4 |
| Tat toxoïde | | 0,2 |
| Tat natif + sérum souris | 2 / J 0 | 1,5 |
| Tat natif + sérum souris | 6 / J 0 | 1,3 |
| Tat natif + sérum souris | 2 / J 40 | 0,3 |
| Tat natif + sérum souris | 6 / J 40 | 1,4 |

Ces résultats montrent que le sérum de souris immunisée (souris 2) contient des anticorps neutralisant la protéine Tat native, ce qui n'est pas le cas pour le sérum d'une souris non immunisée.

De même, si on utilise les anticorps produits par les souris immunisées avec le toxoïde dans le test d'immunosuppression décrit dans l'étude pharmacologique 1) ci-dessus (dilution 1/50), on bloque l'effet immunosuppressif de la protéine Tat native.

Tous ces résultats montrent que le composé de l'exemple 1 est immunogène et permet d'induire une réponse immunitaire qui neutralise la protéine native.

### 3) Immunogénicité du toxoïde IFNα chez la souris, production d'anticorps neutralisants.

Du toxoïde IFNα préparé selon l'exemple 3 a été utilisé pour immuniser des souris. Le protocole d'immunisation est celui classiquement utilisé: les souris sont injectées (en im) avec 100 µl d'émulsion (1:1) en adjuvant complet de Freund contenant 20 µg de produit au jour 0 avec rappel en adjuvant incomplet de Freund de 5 µg aux jours 21, et 35, 45. Le sérum des souris est prélevé aux jours -2, 28 et 50 et analysé par ELISA sur des plaques sensibilisées avec de la protéine IFNa recombinante native (non traitée chimiquement) ou avec du toxoïde. Les sérums ont été testés à une dilution au 1/1000. Les résultats obtenus en densité optique sur 3 souris immunisées (1 à 3) et 3 souris non immunisées (4 à 6) sont présentés dans le tableau suivant:

| | | IFNα natif | IFNα toxoïde |
|---|---|---|---|
| Souris 1 | J -2 | 0,3 | 0,3 |
| | J 28 | 1 | 0,9 |
| | J 50 | 2,1 | 2,1 |
| Souris 2 | J-2 | 0,2 | 0,3 |
| | J 28 | 1,2 | 1,1 |
| | J 50 | 1,9 | 2 |
| Souris 3 | J-2 | 0,4 | 0,4 |
| | J 28 | 0,8 | 0,9 |
| | J 50 | 2 | 2,1 |
| Souris 4 | J -2 | 0,1 | 0,1 |
| | J 28 | 0,2 | 0,2 |
| | J50 | 0,1 | 0,1 |
| Souris 5 | J-2 | 0,2 | 0,2 |
| | J 28 | 0,2 | 0,2 |
| | J50 | 0,2 | 0,3 |
| Souris 6 | J-2 | 0,3 | 0,3 |
| | J 28 | 0,2 | 0,3 |
| | J 50 | 0, 3 | 0,3 |

Ces résultats montrent que les souris immunisées par le toxoïde produisent des anticorps reconnaissant de la même façon la protéine native et le toxoïde. D'autre part cela confirme l'innocuité de l'immunisation par le toxoïde IFNa car les souris ont très bien toléré l'immunisation.

Ces anticorps sont de plus neutralisants. Ceci a été établi par le test classique de virus VSV sur les cellules MDBK. Normalement en ajoutant de 1 à 10 unités d'interféron, on rend les cellules résistantes à la lyse par le virus VSV. En incubant l'IFNα natif avec le sérum de la souris 1 ou 4 (J 0, J 50) dilués au 1/50, la lyse est observée seulement quand l'IFNα natif a été préincubé avec le sérum de la souris 1 de J 50.

Cet exemple montre que le composé de l'exemple 3 est immunogène, avec formation d'anticorps neutralisants.

### 4) Immunogénicité du toxoïde Nef chez la souris.

Du Nef toxoïde préparé selon l'exemple 2 a été utilisé pour immuniser des souris. Le protocole d'immunisation est celui classiquement utilisé: on injecte par voie im aux souris 100 µl d'émulsion (1:1) en adjuvant complet de Freund contenant 20 µg de produit aux jour 0 avec rappel en adjuvant incomplet de Freund de 5 µg aux jours 21, et 35. Le sérum des souris est prélevé aux jours -2, 28 et 40 et analysé par ELISA sur des plaques sensibilisées avec de la protéine Nef recombinante native (non traitée chimiquement) ou avec le toxoïde de l'exemple 2. Les sérums ont été testés à une dilution au 1/1000. Les résultats obtenus exprimés en densité optique sur 3 souris immunisées (1 à 3) et 3 souris non immunisées (4 à 6) sont présentés dans le tableau suivant:

| | | Nef natif | Nef toxoïde |
|---|---|---|---|
| Souris 1 | J-2 | 0,1 | 0,1 |
| | J 28 | 1 | 0,9 |
| | J 50 | 2,1 | 2,1 |
| Souris 2 | J -2 | 0,1 | 0,1 |
| | J 28 | 1,5 | 1,4 |
| | J 50 | 1,9 | 2 |
| Souris 3 | J -2 | 0,1 | 0,1 |
| | J 28 | 0,8 | 0,9 |
| | J 50 | 1,6 | 1,6 |
| Souris 4 | J -2 | 0,1 | 0,1 |
| | J 28 | 0,1 | 0,1 |
| | J 50 | 0,1 | 0,1 |
| Souris 5 | J-2 | 0,1 | 0,1 |
| | J 28 | 0,1 | 0,1 |
| | J 50 | 0,1 | 0,1 |
| Souris 6 | J -2 | 0,1 | 0,1 |
| | J 28 | 0,1 | 0,1 |
| | J 50 | 0,1 | 0,1 |

Ces résultats montrent que les souris immunisées par le toxoïde de l'exemple 2 produisent des anticorps reconnaissant de la même façon la protéine native et le toxoïde. D'autre part cela confirme l'innocuité de l'immunisation par le toxoïde Nef car les souris ont très bien toléré l'immunisation.

### 5) Effet des anticorps de souris immunisées par les toxoïdes Tat et IFNα sur l'infection par VIH-1 in vitro.

On a développé un test d'immunosuppression induite par l'infection VIH-1 in vitro. Ce test est réalisé de la façon suivante :

Des cellules du sang périphérique (PBMCs) d'un sujet sain sont infectées par une souche de VIH-1 lymphotropique (HIV-HTLVIIIB). Après stimulation par Phytohèmagglutinine (PHA) et culture pendant 6 jours en présence d'interleukine-2, les cellules sont irradiées. Les cellules irradiées sont suppressives car capables d'inhiber la prolifération de PBMCs autologues stimulées par des antigènes comme SEB ou PPD. Cet effet immunosuppressif n'est pas lié à du virus résiduel après l'irradiation car les tests de dosage de transcriptase inverse ou d'antigène p24 dans les surnageants sont négatifs. Si on réalise une préparation de cellules sans virus (contrôle), irradiées de la même façon 6 jours après stimulation par PHA, et qu'on les ajoute à des cellules autologues stimulées par SEB ou PPD on n'observe aucune inhibition de la prolifération. L'effet inhibiteur est dû au virus VIH-1.

J F Zagury et al, (Cell Pharmacol AIDS Sciences, 1996, Vol. 3, P. 97-103, A critical role of the Tat and IFNa in the HIV-1 induced immunosuppression leading to AIDS) décrit que la genèse de ces cellules suppressives dépend à la fois de l'IFNa et de la protéine Tat car des anticorps neutralisant ces 2 protéines inhibent la formation de cellules suppressives. Quand dans une expérience de ce type, on a ajouté simultanément les sérums (dilués au 1150) des souris immunisées avec les produits décrits dans les exemples 1 et 3 (Tat toxoïde et IFNa toxoïde), ces sérums bloquent la génération de cellules suppressives. Ce n'est pas le cas avec les sérums de souris non immunisées (contrôle négatif) ou avec les sérums pris avant immunisation. Si on utilise du sérum de souris immunisées avec le Tat toxoïde seul, on ne bloque plus l'immunosuppression. Par contre, quand on utilise du sérum de souris immunisée avec le toxoïde lFNa, l'inhibition de la suppression est de 70%. Si on combine les 2 sérums anti-Tat et anti-IFNa, on bloque complètement la genèse de cellules suppressives. Ces résultats ont été obtenus en utilisant divers lots de virus, notamment des isolats primaires. Cela montre que des anticorps anti-Tat obtenus avec un seul toxoïde peuvent quand même avoir une réactivité croisée.

Ces résultats, décrits par J F Zagury et al. (Cell Pharmacol AIDS Sciences, 1996, Vol. 3, P. 97-103, A critical role of the Tat and IFNa in the HIV-1 induced immunosuppression leading to AIDS), confirment l'effet inhibiteur combiné de Tat et de l'IFNα vis à vis de l'état immunosuppressif chez les patients au stade SIDA. lls confirment de plus le rôle initiateur de Tat et le rôle déterminant de l'IFNα, dans cette immunosuppression (voir publication D. Zagury et al. (IFN and Tat involvement in the immunosuppression of uninfected T cells and C-C chemokine decline in AIDS, Proc. Natl. Acad. Sci. USA, 1998, sous presse). Les résultats de ces travaux montrent la nécessité de lutter contre la protéine Tat extracellulaire par l'induction d'une réponse anticorps neutralisante en intervenant, si possible, dès la production du Tat par l'induction d'une réponse immunitaire cellulaire.

### 6. Immunogénicité et innocuité des toxoïdes Tat et IFNα combinés ou non chez l'homme. Compatibilité avec les chimiothérapies_{.}

Les Tableaux 1, 2, 3 décrivent les résultats obtenus lors d'un essai de phase 1 utilisant les toxoïdes des exemples 1 et 3 en émulsion 50-50 avec de l'ISA 51 (SEPPIC) : volume d'1 ml contenant 100 µg d'agent actif selon l'invention. Les réponses à l'IFNa toxoïde (données non montrées ici) sont similaires à celles déjà décrites dans les publications de Gringeri et al. dans JAIDS 1994, Vol. 7, 978-988 et 1996, Vol. 13, 55-67 sur un toxoïde réalisé par formolation.

Tableau 1: description des patients inclus dans l'essai de phase 1.

Tableau 2: paramètres biologiques au temps initiaux et finaux (en général sur 6 mois).

Tableau 3: analyse de la réponse spécifique anti-Tat.

**Tableau 1 Démographie de patients immunisés contre le Tat de HIV-1, âge au moment de la séroconversion et traitement antirétroviral**

| Code | Age | Sexe | Facteur de risque | Age au moment de la séroconversion | Traitement antiviral | | lmmunisation Anti-IFNα |
|---|---|---|---|---|---|---|---|
| | | | | | | avec protéinase | |
| 1 | 25 | M | Hémophilie | 12 | O | O | O |
| 2 | 39 | M | Hémophilie | 26 | N | N | N |
| 3 | 33 | F | Partenaire sexuel | 26 | O | O | O |
| 4 | 38 | M | Hémophilie | 23 | N | N | O |
| 5 | 25 | M | Hémophilie | 12 | N | N | N |
| 6 | 31 | M | Hémophilie | 19 | O | N | N |
| 7 | 19 | M | Hémophilie | 7 | N | N | O |
| 8 | 35 | M | Hémophilie | 22 | O | N | O |
| 9 | 26 | M | Hémophilie | 15 | N | N | N |
| 10 | 38 | M | Hémophilie | 26 | N | N | N |
| 11 1 | 26 | M | Hémophilie | 14 | O | O | N |
| 12 | 33 | M | Hémophilie | 20 | O | O | N |
| 13 | 40 | F | Partenaire sexuel | 36 | N | N | N |
| 14 | 26 | F | Partenaire sexuel | 20 | N | N | O |

**Tableau 2 Comptage des cellules CD4 en valeur absolue, virémie plasmatique VIH-1 et antigénémie chez 14 patients séropositifs immunisés avec les produits des exemples 1 ou 3**

| Code | Suivi en mois | Comptage des cellules de CD4 en cellules/mm³ | | Virémie VIH-1 | | Antigènes anti-VIH | |
|---|---|---|---|---|---|---|---|
| | | Avant | Après | Basal | Après | Basal | Après |
| 1 | 8 | 284 (28,4 %) | 345 (34,5 %) | 5,75 | 5,71 | 11 | 4 |
| 2 | 8 | 453(16,8%) | 534(18,4%) | 2,06 | 2,15 | 32 | 3 |
| 3 | 12 | 227 (28,4 %) | 345 (34,5 %) | 0,35 | <0,005 | 17 | 4 |
| 4 | 3 | 287 (22,1 %) | 252 (21,0 %) | 0,49 | 0,70 | 3 | 3 |
| 5 | 5 | 282 (28,2 %) | 319 (22,8 %) | 3,34 | 0,91 | 4 | 4 |
| 6 | 9 | 224 (11,8 %) | 294 (12,8 %) | 5,23 | 3,90 | 3 | 3 |
| 7 | 3 | 313 (24,1 %) | 443 (23,3 %) | 5,39 | 5,55 | 4 | 4 |
| 8 | 4 | 275(30,6%) | 314 (31,4 %) | 4,12 | 3,18 | 5 | 4 |
| 9 | 6 | 274 (8,3 %) | 386 (9,9 %) | 5,08 | 5,74 | 4 | 5 |
| 10 | 8 | 295 (22,7 %) | 372 (26,6 %) | 4,02 | 3,9 | 5 | 4 |
| 11 | 4 | 219 (16,9 %) | 235 (23,5 %) | 4,01 | <1 | 8 | 3 |
| 12 | 12 | 272 (20,9 %) | 340 (30,9 %) | 4,74 | 2,30 | n.a. | 4 |
| 13 | 9 | 435 (33,5 %) | 478 (28,1 %) | 4,30 | 4,07 | 3 | 4 |
| 14 | 3 | 257 (13,5 %) | 274 (13,7% | 2,05 | 2,90 | 3 | 3 |

**Tableau 3 lmmunogénicité de l'immunisation anti-Tat de VIH-1 chez les patients VIH-1 séropositifs**

| Code patient | Immunisation type (AB) | Réponse anti-Tat anticorps | Anticorps anti-Tat (en unités de Densité optique) | | Réponse HSR | Réponse CMI |
|---|---|---|---|---|---|---|
| | | | Basal | Pic | | |
| 1 | B | 0 | 0,240 | 1,230 | n.a | n.a |
| 2 | A | 0 | 0,104 | 1,526 | n.a | POS |
| 3 | B | O | 0,217 | 0,537 | NEG | NEG |
| 4 | B | O | 0,214 | 1,063 | POS | n.a |
| 5 | A | O | 0,446 | 1,612 | n.a | n.a |
| 6 | A | O | 0,213 | 0,664 | NEG | NEG |
| 7 | A | O | 0,204 | 1,139 | n.a | n.a |
| 8 | B | O | 0,104 | 1,045 | POS | POS |
| 9 | A | O | 0,422 | 0,812 | NEG | n.a |
| 10 | B | O | 0,125 | 1,492 | n.a | n.a |
| 11 | A | O | 0,252 | 0,682 | NEG | n.a |
| 12 | A | O | 0,410 | 2,219 | POS | POS |
| 13 | A | O | 0,162 | 0,508 | POS | POS |
| 14 | B | O | 0,120 | 2,012 | n.a | n.a |

| | | | | | | |
|---|---|---|---|---|---|---|
| HSR : Hypersensibilité retardée | | | | | | |
| CMI : lmmunité à médiation cellulaire. | | | | | | |

## Revendications

1. Une protéine ou fragment de protéine de 8 à 110 acides aminés, **caractérisé en ce qu'**ils sont carboxyméthyles par réaction avec l'acide iodoacétique et **en ce qu'**il s'agit d'une protéine de régulation virale ou fragment de protéine de régulation virales

2. Une protéine ou un fragment selon la revendication 1, **caractérisé en ce que** le virus est VIH 1 ou VIH 2.

3. Une protéine ou un fragment selon la revendication 1 ou 2, **caractérisé en ce qu'**il est une protéine de régulation virale ou un fragment de protéine de régulation virale.

4. Une protéine ou un fragment selon la revendication 1 ou 2, **caractérisé en ce qu'**il provient de Tat.

5. Un procédé de préparation d'une protéine ou d'un fragment tel que défini à la revendication 1, **caractérisé en ce que** l'on soumet ladite protéine ou ledit fragment à une carboxyméthylation par réaction avec l'acide iodoacétique, pour obtenir le composé attendu que l'on isole.

6. Un procédé selon la revendication 5, **caractérisé en ce que** ladite protéine ou ledit fragment se présente sous forme fusionnée à un marqueur (FP) lors de la carboxyméthylatian.

7. Un Procédé selon la revendication 6, **caractérisé en ce que** le marqueur est clivé après purification.

8. Un procédé selon la revendication 6 ou 7, **caractérisé en ce que** le marqueur comprend un composé peptidique comprenant plusieurs histidines.

9. Une protéine ou fragment selon la revendication 1, pour son utilisation dans une méthode de traitement thérapeutique du corps humain ou animal.

10. Une protéine ou fragment de 8 à 110 acides aminés de la protéine Tat de VIH 1 carboxyméthylé par réaction avec l'acide iodoacétique pour son utilisation dans une méthode de traitement thérapeutique du corps humain ou animal.

11. Une composition pharmaceutique, **caractérisée en ce qu'**elle renferme à titre de principe actif l'un au moins des protéines ou fragments carboxyméthylés tels que définis à la revendication 1, ainsi qu'un excipient pharmaceutiquement acceptable.

12. Une composition pharmaceutique, **caractérisée en ce qu'**elle renferme à titre de principe actif l'un au moins des protéines ou fragments tels que définis à l'une quelconque des revendications 2 à 4 ainsi qu'un excipient pharmaceutiquement acceptable.

13. Un vaccin, **caractérisé en ce qu'**il renferme à titre de principe actif l'un au moins des protéines ou fragments tels que définis à l'une des revendications 1 à 4.

14. Une protéine **caractérisée en ce qu'**elle est obtenue par carboxyamidométhylation par réaction avec l'iodoacétamide et **en ce qu'**il s'agit d'une protéine de régulation virale.

15. Une protéine selon la revendication 14, **caractérisée en ce que** le virus est VIH 1 ou VIH 2.

16. Une protéine selon l'une des revendications 14 ou 15, **caractérisée en ce qu'**elle consiste en la protéine Tat.

17. Procédé de préparation d'une protéine selon l'une des revendications 14 à 16, **caractérisé en ce que** l'on soumet ladite protéine à une carboxyamidométhylation par réaction avec l'iodoacétamide, pour obtenir le composé attendu que l'on isole.

18. Procédé selon la revendication 17, **caractérisé en ce que** la protéine se présente sous forme fusionnée à un marqueur (FP) lors de la carboxyamidométhylation.

19. Procédé selon la revendication 18, **caractérisé en ce que** - le marqueur est clivé après purification.

20. Procédé selon l'une des revendications 18 et 19, **caractérisé en ce que** le marqueur comprend un composé peptidique comprenant plusieurs histidines.

21. Un fragment de protéine de 8 à 110 acides aminés, **caractérisé en ce qu'**il est obtenu par carboxyamidométhylation par réaction avec l'iodoacétamide, **en ce qu'**il s'agit d'un fragment d'une protéine de régulation virale, ledit fragment se présentant sous forme fusionnée à un marqueur (FP) lors de la carboxyamidométhylation.

22. Fragment selon la revendication 21, **caractérisé en ce que** le marqueur comprend un composé peptidique comprenant plusieurs histidines.

23. Une protéine ou un fragment de protéine selon l'une des revendications 14 à 16 et 21 à 22, pour son utilisation dans une méthode de traitement thérapeutique du corps humain ou animal.

24. Une composition pharmaceutique, **caractérisée en ce qu'**elle renferme à titre de principe actif au moins une protéine ou un fragment de protéine tels que définis dans l'une des revendications 14 à 16 et 21 à 22, ainsi qu'un excipient pharmaceutiquement acceptable.

25. Un vaccin, **caractérisé en ce qu'**il renferme à titre de principe actif au moins une protéine ou un fragment de protéine tels que définis dans l'une des revendications 14 à 16 et 21 à 22.

26. Un vaccin selon la revendication 25, **caractérisé en ce qu'**il comprend un excipient pharmaceutiquement acceptable.

27. Un vaccin selon la revendication 26, **caractérisé en ce que** l'excipient pharmaceutiquement acceptable consiste en un adjuvant.

## Claims

1. A protein or a protein fragment having 8 to 110 aminoacids, **characterized in that** it is carboxymethylated by reaction with iodoacetic acid and it is a viral regulation protein or a fragment of a viral regulation protein.

2. A protein or a fragment according to claim 1, **characterized in that** the virus is HIV-1 or HIV-2.

3. A protein or a fragment according to claim 1 or 2, **characterized in that** it is a viral regulation protein or a fragment of a viral regulation protein.

4. A protein or a fragment according to claim 1 or 2, **characterized in that** it comes from Tat.

5. Method for preparing a protein or a fragment as defined in claim 1, **characterized in that** said protein or said fragment is submitted to a carboxymethylation step by reaction with iodoacetic acid so as to obtain the expected compound which is isolated.

6. Method according to claim 5, **characterized in that** said protein or said fragment is present as a fused form with a marker (EP) upon the carboxymethylation.

7. Method according to claim 6, **characterized in that** the marker is cleaved after purification.

8. Method according to claim 6 or 7, **characterized in that** the marker comprises a peptidic compound comprising several histidines.

9. A protein or a fragment according to claim 1, for its use in a therapeutic treatment method for the human or animal body.

10. A protein or a fragment having 8 to 110 aminoacids of HIV-1 Tat protein carboxymethylated by reaction with iodoacetic acid for its use in a therapeutic treatment method for the human or animal body.

11. A pharmaceutical composition **characterized in that** it comprises as an active ingredient one at least of carboxymethylated proteins or fragments as defined in claim 1, as well as a pharmaceutically acceptable excipient.

12. A pharmaceutical composition **characterized in that** it comprises as an active ingredient one at least of the proteins or fragments as defined in any one of claims 2 to 4, as well as a pharmaceutically acceptable excipient.

13. A vaccine **characterized in that** it comprises as an active ingredient one at least of the proteins or fragments as defined in any one of claims 1 to 4.

14. A protein **characterized in that** it is obtained by carboxymethylation by reaction with iodoacetamide and **in that** it is a viral regulation protein.

15. A protein according to claim 14, **characterized in that** the virus is HIV-1 or HIV-2.

16. A protein according to one of claims 14 or 15, **characterized in that** it is Tat protein.

17. Method for preparing a protein according to one of claims 14 to 16, **characterized in that** said protein is submitted to a carboxymethylation step by reaction with iodoacetamide so as to obtain the expected compound which is isolated.

18. Method according to claim 17, **characterized in that** said protein is present as a fused form with a marker (FP) upon the carboxymethylation.

19. Method according to claim 18, **characterized in that** the marker is cleaved after purification.

20. Method according to one of claims 18 and 19, **characterized in that** the marker comprises a peptidic compound comprising several histidines.

21. A protein fragment having 8 to 110 aminoacids, **characterized in that** it is obtained by carboxymethylation by reaction with iodoacetamide, **in that** it is a viral regulation protein fragment, said fragment is present as a fused form with a marker (FP) upon the carboxymethylation.

22. A fragment according to claim 21, **characterized in that** the marker comprises a peptidic compound comprising several histidines.

23. A protein or a protein fragment according to one of claims 14 to 16 and 21 to 22 for its use in a therapeutic treatment method for the human or animal body.

24. A pharmaceutical composition, **characterized in that** it comprises as an active ingredient at least a protein or a protein fragment, as defined in one of claims 14 to 16 and 21 to 22, as well as a pharmaceutically acceptable excipient.

25. A vaccine **characterized in that** it comprises as an active ingredient at least a protein or a protein fragment as defined in one of claims 14 to 16 and 21 to 22.

26. A vaccine according to claim 25, **characterized in that** it comprises a phamaceutically acceptable excipient.

27. A vaccine according to claim 26, **characterized in that** the phamaceutically acceptable excipient is an adjuvant.

## Patentansprüche

1. Protein oder Proteinfragment von 8 bis 110 Aminosäuren, **dadurch gekennzeichnet, dass** sie durch Reaktion mit Iodessigsäure carboxymethyliert sind und dass es sich um ein virales Regulatorprotein oder ein Fragment eines viralen Regulatorproteins handelt.

2. Protein oder Fragment gemäß Anspruch 1, **dadurch gekennzeichnet, dass** es sich bei dem Virus um HIV-1 oder HIV-2 handelt.

3. Protein oder Fragment gemäß Anspruch 1 oder 2, **dadurch gekennzeichnet, dass** es sich um ein virales Regulatorprotein oder ein Fragment eines viralen Regulatorproteins handelt.

4. Protein oder Fragment gemäß Anspruch 1 oder 2, **dadurch gekennzeichnet, dass** es von Tat stammt.

5. Verfahren zur Herstellung eines Proteins oder Fragments, wie es in Anspruch 1 definiert wurde, **dadurch gekennzeichnet, dass** man das Protein oder Fragment einer Carboxymethylierung durch Reaktion mit Iodessigsäure unterzieht, so dass man die erwartete Verbindung erhält, die man dann isoliert.

6. Verfahren gemäß Anspruch 5, **dadurch gekennzeichnet, dass** das Protein oder Fragment während der Carboxymethylierung in einer Form vorliegt, in der es mit einem Marker fusioniert ist (FP).

7. Verfahren gemäß Anspruch 6, **dadurch gekennzeichnet, dass** der Marker nach der Reinigung abgespalten wird.

8. Verfahren gemäß Anspruch 6 oder 7, **dadurch gekennzeichnet, dass** der Marker eine Peptidverbindung umfasst, die mehrere Histidinreste umfasst.

9. Protein oder Fragment gemäß Anspruch 1 zur Verwendung in einem Verfahren der therapeutischen Behandlung des menschlichen oder tierischen Körpers.

10. Protein oder Fragment von 8 bis 110 Aminosäuren des Tat-Proteins von HIV-1, carboxymethyliert durch Reaktion mit Iodessigsäure zur Verwendung in einem Verfahren der therapeutischen Behandlung des menschlichen oder tierischen Körpers.

11. Pharmazeutische Zusammensetzung, **dadurch gekennzeichnet, dass** sie als Wirkstoff wenigstens eines der carboxymethylierten Proteine oder Fragmente, wie sie in Anspruch 1 definiert sind, sowie einen pharmazeutisch annehmbaren Arzneimittelhilfsstoff umfasst.

12. Pharmazeutische Zusammensetzung, **dadurch gekennzeichnet, dass** sie als Wirkstoff wenigstens eines der Proteine oder Fragmente, wie sie in einem der Ansprüche 2 bis 4 definiert sind, sowie einen pharmazeutisch annehmbaren Arzneimittelhilfsstoff umfasst.

13. Impfstoff, **dadurch gekennzeichnet, dass** er als Wirkstoff wenigstens eines der Proteine oder Fragmente umfasst, wie sie in einem der Ansprüche 1 bis 4 definiert sind.

14. Protein, **dadurch gekennzeichnet, dass** es durch Carboxyamidomethylierung durch Reaktion mit Iodacetamid erhalten wird und dass es sich um ein virales Regulatorprotein handelt.

15. Protein gemäß Anspruch 14, **dadurch gekennzeichnet, dass**, es sich bei dem Virus um HIV-1 oder HIV-2 handelt.

16. Protein gemäß einem der Ansprüche 14 oder 15, **dadurch gekennzeichnet, dass** es sich um das Tat-Protein handelt.

17. Verfahren zur Herstellung eines Proteins gemäß einem der Ansprüche 14 bis 16, **dadurch gekennzeichnet, dass** man das Protein einer Carboxyamidomethylierung durch Reaktion mit Iodacetamid unterzieht, so dass man die erwartete Verbindung erhält, die man dann isoliert.

18. Verfahren gemäß Anspruch 17, **dadurch gekennzeichnet, dass** das Protein während der Carboxyamidomethylierung in einer Form vorliegt, in der es mit einem Marker fusioniert ist (FP).

19. Verfahren gemäß Anspruch 18, **dadurch gekennzeichnet, dass** der Marker nach der Reinigung abgespalten wird.

20. Verfahren gemäß einem der Ansprüche 18 und 19, **dadurch gekennzeichnet, dass** der Marker eine Peptidverbindung umfasst, die mehrere Histidinreste umfasst.

21. Proteinfragment von 8 bis 110 Aminosäuren, **dadurch gekennzeichnet, dass** es durch Carboxyamidomethylierung durch Reaktion mit Iodacetamid erhalten wird und dass es sich um ein Fragment eines viralen Regulatorproteins handelt, wobei das Fragment während der Carboxyamidomethylierung in einer Form vorliegt, in der es mit einem Marker fusioniert ist (FP).

22. Fragment gemäß Anspruch 21, **dadurch gekennzeichnet, dass** der Marker eine Peptidverbindung umfasst, die mehrere Histidinreste umfasst.

23. Protein oder Fragment gemäß einem der Ansprüche 14 bis 16 und 21 bis 22 zur Verwendung in einem Verfahren der therapeutischen Behandlung des menschlichen oder tierischen Körpers.

24. Pharmazeutische Zusammensetzung, **dadurch gekennzeichnet, dass** sie als Wirkstoff wenigstens ein Protein oder Proteinfragment, wie sie in einem der Ansprüche 14 bis 16 und 21 bis 22 definiert sind, sowie einen pharmazeutisch annehmbaren Arzneimittelhilfsstoff umfasst.

25. Impfstoff, **dadurch gekennzeichnet, dass** er als Wirkstoff wenigstens ein Protein oder Proteinfragment umfasst, wie sie in einem der Ansprüche 14 ' bis 16 und 21 bis 22 definiert sind.

26. Impfstoff gemäß Anspruch 25, **dadurch gekennzeichnet, dass** er einen pharmazeutisch annehmbaren Arzneimittelhilfsstoff umfasst.

27. Impfstoff gemäß Anspruch 26, **dadurch gekennzeichnet, dass** es sich bei dem pharmazeutisch annehmbaren Arzneimittelhilfsstoff um ein Adjuvans handelt.
